# FASCICULE DE BREVET EUROPEEN

(11) **EP 1 498 074 B1**
(45) Date de publication et mention de la délivrance du brevet: **08.06.2011**
(21) Numéro de dépôt: 02796779.3
(22) Date de dépôt: 09.12.2002
(51) Int. Cl.: A61B 17/02

(54) **ECARTEURS MAMMAIRES SPECIFIQUES POUR L'INTRODUCTION DE PROTHESE DANS LA MAMMOPLASTIE D'AUGMENTATION**
SPEZIFISCHE BRUSTRETRAKTOREN FÜR DIE EINFÜHRUNG VON IMPLANTATEN IN DER AUGMENTATIONSMAMMOPLASTIE
SPECIFIC MAMMARY RETRACTORS FOR THE INSERTION OF IMPLANTS IN AUGMENTATION MAMMOPLASTY

(30) Priorité: 19.12.2001 ES 200102835
(43) Date de publication de la demande: 19.01.2005
(73) Titulaire: Quintas Díez, Ramón, 22004 Huesca (ES)
(72) Inventeur: Quintas Díez, Ramón, 22004 Huesca (ES)
(86) Numéro de dépôt international: PCT/ES2002/000585
(87) Numéro de publication internationale: WO 2003/051203

(56) Documents cités:
- WO-A1-99/05973
- GB-A- 2 133 694
- JP-A- 7 124 170
- US-A- 3 701 348
- US-A- 3 729 006
- US-A- 4 151 838
- US-A- 4 934 352
- US-A- 5 351 680

## Description

### SECTEUR DE LA TECHNIQUE.

La présente invention fait référence à deux séparateurs chirurgicaux égaux grâce auxquels on facilite l'introduction des prothèses mammaires dans la plastie mammaire d'augmentation. On raccourcit le temps chirurgical dans cette manoeuvre, et on evitent les risques inhérents qui sont posés avec les séparateurs employés jusqu'à présent, en apportant une série d'avantages.

### ÉTAT DE LA TECHNIQUE.

Sont connus divers séparateurs chirurgicaux qui ont été conçus pour l'application en chirurgie générale fondamentalement, comme les séparateurs de Farabeuf, de Collin, de Parker, de Lagenveck ou de Roux ou ceux décrit dans le document US 5 351 680, et qui ne sont pas spécifiques pour la manoeuvre d'introduction des prothèses mammaires.

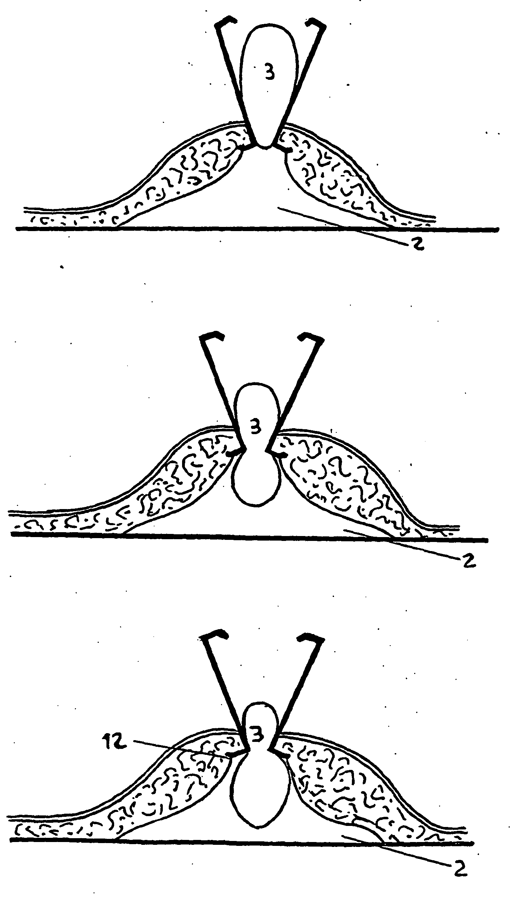

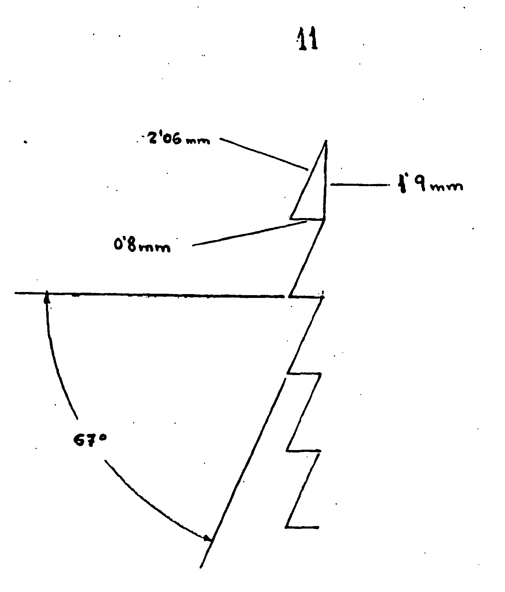

Les séparateurs de Farabeuf sont utilisés plus fréquemment dans l'actualité dans la plastie mammaire d'augmentation, en implicant une grande difficulté tant pour l'opérateur comme pour l'assistant à l'heure d'introduire la prothèse, sur tout si celles-ci sont grandes et/ou l'aréole mammaire est moyenne ou petite. Avec ces séparateurs ne sont pas exempts quelques risques comme la rupture de la prothèse ou les déchirures de peau dans les bords chirurgicaux. Les faces de ces séparateurs sont lisses, ce pourquoi quand on introduit la prothèse elle glisse bien par le biais d'eux, mais avec la même facilité elle glisse aussi vers

le haut, parce que ces séparateurs traditionnels ne croient aucune résistance. De même, quand peu à peu on introduit la prothèse avec ces séparateurs, figures 5,.6 et 7 (1), commence une augmentation progressive de la pression atmosphérique (Pr. Atm.) dans l'espace rétroglandulaire (2) qui diminue, et il n'y eu pas possibilité d'échappement d'air, parce que la même prothèse (3) fait de bouchon dans la coupole de la poitrine (4). Cet accroissement de la Pr. Atm. dans l'espace rétroglandulaire fait pousser la prothèse vers l'extérieur (5) et (6), en augmentant la difficulté dans la manoeuvre d'introduction de la prothèse.

Pour éviter ces problèmes techniques existants jusqu'à présent, le titulaire de la présente invention, D Ramón Quintas Díez, conçoit des séparateurs nouveaux et spécifiques pour l'introduction des prothéses dans la plastie mammaire dàugmentation, dont les caractéristiques sont l'objet de cette invention et qui met à décrire dans l'explication, description des dessins, la réalisation et les revendications.

### EXPLICATION DE L'INVENTION.

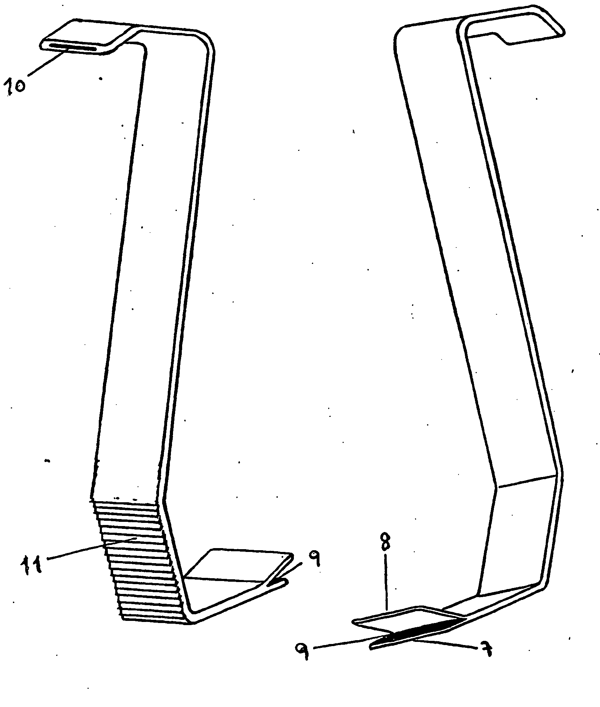

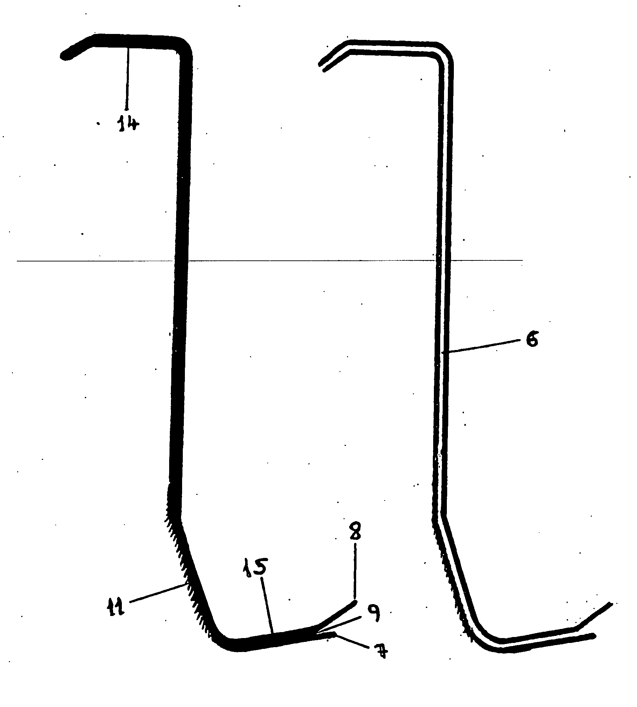

Chaque séparateur, qui a la forme d'un deux étiré, est creux dans tout son intérieur, en y ayant une solution de continuité complète d'un extrême à l'autre, figure 4. Dans l'extrême inférieur on observe une rainure, figure 3 (9) et figure 2 (9), par où entrera l'air soumis à pression dans l'espace rétroglandulaire, en parcourant tout le trajet creux du séparateur jusqu'à l'autre rainurede sortie de l'extême supérieur, figure 1 (10). De cette manière nous parviendrons à maintenir toujours la même Pr. Atm. dans l'espace rétroglandulaire (101325 Pa, 760 mm de Hg.), et conséquemment quand on introduit la prothèse avec les doigts nous ne provoquerons aucune augmentation de pression dans l'espace rétroglandulaire, qui fait freiner la prothèse vers l'intérieur.

L'extrême inférieur du séparateur dispose d'un cil, figure 2 (8), qui fait de visière pour protéger la rainure de c'extrême, pour que ne soit pas obstruée par la base de la glande mammaire quand celle-ci est tenue par le séparateur, figure 7 (12). De se produire ceci nous perdrions l'effet d'échappement de l'air soumis à pression dans l'espace rétroglandulaire, par bouchage. Cette rainure est aussi protégée par un autre cil inférieur, figure 2 (7).

La partie inférieure de la face convexe du séparateur n'est pas lisse, parce qu'est striée sur base de petites coupes transversales en biseau, figure 1 (11) et figure 8. Cette caractéristique fait que la prothèse mammaire glisse sans difficulté vers l'intérieur de l'espace rétroglandulaire quand nous l'introduirons avec les doigts, mais on provoque une fixation ou frei quand la prothèse a tendance à monter pandant les mouvements d'oscillation de la manoeuvre digitaled'introduction, faite qui n'arrive pas avec les séparateurs employés actuellement, par être ces lisses.

### BRÈVE DESCRIPTION DES DESSINS.

La figure 1 montre le séparateur vu par sa face convexe et en position oblique, où on distingue la rainure supérieure (10) et lestrié inférieur (11).
La figure 2 présente le séparateur vu par sa face concave et aussi en position oblique, où on montrent les cils (8) et (7), et la rainure inférieure (9).
La figure 3 est le séparateur vu latéralement ou de profil pour montrer les quatre segments, les angles et les dimensions réelles (échelle 1:1).
La figure 4 c'est la même vue latérale, mais avec une coupe ou section longitudinale complète du séparateur, pour montrer la solution de continuité complète du creux intérieur, en voyant comment on communiquent parfaitement les deux extrêmes du séparateur.
Les figures 5, 6 et 7 montrent trois images pendant l'introduction d'une prothèse mammaire (3), avec les séparateurs traditionnels (1). La prothèse entre par le glande mammaire (13) coupée longitudinalement pour visualiser son intérieur ou espace rétroglandulaire (2).
La figure 8 montre latéralement le strié, comme petites dents, avec ses angles et dimensions (échelle 10:1).

### MODE DE RÉALISATION DE L'INVENTION.

Chaque séparateur mammaire (sont deux unités égales), est une pièce non articulée, non flexible et très dure, parce qu'il est d'acier inoxydable.

Nous pouvons diviser le séparateur dans quatre segments, figure 3: deux segments horizontaux (14) et (15), et deux autres verticales (16) et (17).

Le segment (14) forme un angle droit (18) avec le segment (16). Mesure 3 cm en comptant un petit doublage dans son extrême libre de 8 mm. Dans l'épaisseur de son extrême libre on observe la rainure de sortie d'air, figure 1 (10). Il est lisse par les deux faces. La face inférieure on placera sur le premier doigt de l'assistant.

Le segment (16) forme un angle de 163° (19) avec le segment (17) par sa face concave. Il mesure 12 cm de longueur. Il est aussi il est lisse par les deux faces.

Le segment (17) forme angle droit (20) avec le segment (15) par sa face concave. Il mesure 3.3 cm de longueur. Sa face convexe est striée ou ébréchée, et par elle, avec la face striée de l'autre séparateur, on glissera la prothèse. La face concave est lisse, et c'est celle qui séparera le tissu glandulaire de la coupole mammaire, avec l'autre séparateur, pour créer un tunnel par où on introduira la prothèse.

Le segment (15) est lisse par les deux faces. Il mesure 2.7 cm par sa face inférieure ou convexe, et 3,4 cm par sa face supérieure ou concave, y avec le cil (8) de 1 mm d'épaisseur et 1 cm de longueur. Le cil forme un angle de 154° (21) par sa face concave ou supérieure. L'extrême distal de la face inférieure, cil (7), mesure 5 mm de longueur et 1 mm d'épaisseur. Ces deux cils (8) et (7), forment un angle de 25° (29), et dans sa confluence on trouve la rainure inférieure (9), de 1 mm d'épaisseur. La face supérieure du segment (15) sujet la base de la glande mammaire, et le cil (8) fait que le tissu mammaire ne puisse pas obturer la rainure inférieure. Le cil (8) a aussi une fonction de protection de la cannelure, en empêchant qu'entrent des restes de tissus ou de liquides.

La longueur totale du séparateur est de 15 cm, sa largeur est de 2.5 cm et son épaisseur est de 3 mm, en mesurant chaque face 1 mm d'épaisseur, et le creux 1 mm, figure 4.

Ils seront indépendants de l'objet de l'invention les dimensions et d'autres détails accessoires qui peuvent être présentés, pourvu qu'ils n'affectent pas son integridad.

### APPLICATION INDUSTRIELLE.

Par la nature de l'invention est viable une application industrielle. Les deux séparateurs mammaires ont été fabriqués de manière individuelle (prototypes) dans une entreprise de métaux et soudures de Huesca (L'Espagne) et ont été mis en pratique dans des nombreuses plasties mammaires d'augmentation depuis le 30 novembre 2001 avec les résultats attendus de l'invention.

Avec des fins commerciales, ces séparateurs mammaires on fabriquent déjà en série avec l'entreprise allemande de fabrication d'instruments chirurgicaux "Schreiber Instrumente".

## Revendications

1. Séparateur mammaire spécifique pour l'introduction des prothèses dans la plastie mammaire d'augmentation, avec la forme d'un deux allongé, en acier inoxidable, non articulé, **caractérisé en ce que** chaque extrême du séparateur comprend une rainure (9, 10) et **en ce que** le séparateur a une configuration creuse dans toute son longueur (6) de continuité complète entre les dites deux rainures (9; 10) qui font communiquer l'espace rétroglandulaire mammaire (2) avec l'extérieur, de manière à maintenir celui-là toujours avec la même pression atmosphérique au moment d'introduction des dites prothèses.

2. Séparateur mammaire spécifique pour l'introduction des prothèses dans la plastie mammaire d'augmentation, selon le revendication 1, **caractérisé en ce que** la partie inférieure de chaque séparateur de la face convexe (11) de chaque séparateur est striée ou ébréchée sur base de petites coupes transversales en biseau, en facilitant et en raccourcissant la manoevre d'introduction des prothèses mammaires (3).

3. Séparateur mammaire spécifique pour l'introduction des prothèses dans la plastie mammaire d'augmentation, selon le revendication 1, **caractérisé en ce que** le séparateur comprend un segment horizontal supérieur (14) et un segment horizontal inférieur (15), et que le segment horizontal supérieur (14) est contraire au sens du segment horizontal inférieur (15), pour que celui-là s'appuie sur le premier doigt de l'assistant.

4. Séparateur mammaire spécifique pour l'introduction des prothèses dans la plastie mammaire d'augmentation, selon le revendication 1, **caractérisé par** le fait d'avoir deux cils (8, 7), qui protègent la rainure inférieure (9), en empêchant que celle-ci soi bouchée par le tisù glandulaire (12).

## Claims

1. Specific mammary retractor for the insertion of implants in augmentation mammoplasty are like and extended two in shape. These new retractors have been made of non-articulated stainless steel being hollow along the grooves disposed at the retractor ends (9, 10), allowing to communicate the retroglandular region (2) when with the exterior, and consequently maintaining all time the same atmospheric pressure within the retrogladular region when the implant is inserted (3).

2. Specific mammary retractor fot the insertion of implants in augmentation mammoplasty as claimed in claim 1, wherein the lower portion of every retractor convex face (11) is striated or nicked by little transversal bevel cuts with high sloping in one direction only. Such characteristic ensures that the peosthesis can slide easily when inserting it in inner, making easier the manoeuvre for the insertion of implants (3) and being greatly simplified due to reduction of operation time.

3. Specific mammary retractors for the insertion of implants in augmentation mammoplasty as claimed in claim 1, wherein the upper horizontal segment (14), is disposed in the opposite direction to the lower horizontal segment thereof (15). The reason for that is this way the surgical assistant can hold the retractor with his thumb managing a more effective manoeuvre.

4. Specific mammary retractors for the insertion of implants in augmentation mammoplasty as claimed in claim 1, wherein the two flanges (8, 7), are protecting the lower groove (9), prevent it from being sealing off by glandular tissure (12).

## Patentansprüche

1. Spezifischer Brustretraktor für die Einführung von Implantaten in der Augmentationsmammoplastie, in der Form einer länglichen Zwei, aus Edelstahl, nicht gegliedert. Da der Brustretraktor in voller Länge hohl gestaltet ist (6), entsteht zwischen beiden Schlitzen am Ende jedes Trenners (9,10) eine komplette Kontinuitätstrennung. Die Schlitze ermöglichen den retroglandularen Brustraum (2) nach außen zu verbinden, sodass er (2) bei der Einführung der Prothese (3) denselben Luftdruck hält.

2. Spezifischer Brustretraktor für die Einführung von Implantaten in der Augmentationsmammoplastie. Gemäß Anspruch 1 ist der Brustretraktor **dadurch gekennzeichnet, dass** der untere Teil der Konvexseite (11) mittels kleiner abgeschrägter Querschnitte gerippt oder ausgezackt ist, sodass der Handgriff zur Einführung der Prothese (3) leichter und kürzer wird.

3. Spezifischer Brustretraktor für die Einführung von Implantaten in der Augmentationsmammoplastie. Gemäß Anspruch 1 ist der Brustretraktor **dadurch gekennzeichnet, dass** jeder Trenner ein horizontales Obersegment (14) und ein horizontales Untersegment (15) hat. Das horizontale Obersegment (14) liegt in umgekehrter Richtung zum horizontalen Untersegment (15), damit das Obersegment auf den Daumen des Helfers aufstützt.

4. Spezifischer Brustretraktor für die Einführung von Implantaten in der Augmentationsmammoplastie. Gemäß Anspruch 1 ist der Brustretraktor **dadurch gekennzeichnet, dass** er zwei angebrachte Flansche (8,7) hat, welche den unteren Schlitz (9) schützen. Somit wird ein Verschluss des unteren Schlitzes durch das Drüsengewebe (12) verhindert.
